# EUROPEAN PATENT APPLICATION

(11) **EP 2 559 399 A1**
(43) Date of publication of application: **20.02.2013**
(21) Application number: 10849909.6
(22) Date of filing: 15.11.2010
(51) Int. Cl.: A61C 19/04, G01N 25/00

(54) **THERMAL TESTER FOR DENTAL CLINIC**

(30) Priority: 16.04.2010 KR 20100035072
(71) Applicant: B&L Biotech Co., Ltd., 1136-1 Sanbon-dong Gunpo-si, Gyeonggi-do 435-040 (KR)
(72) Inventor: LEE, In Hwan, Gunpo-si Gyeonggi-do 435-040 (KR)
(74) Representative: Tischner, Oliver
(86) International application number: PCT/KR2010/008026
(87) International publication number: WO 2011/129505

(57) **Abstract**

Disclosed is a thermal tester for a dental clinic for accurately measuring dental pulp thermal sensitivity by rapidly delivering heat at a predetermined temperature to a tooth. The thermal tester for a dental clinic of the present invention comprises: a tester body having on one side a protruding heat transfer terminal provided with a contact surface that comes into contact with the tooth; and a heating unit for heating the heat transfer terminal, wherein the heating unit includes a first heating element and a second heating element, which, in a mutually electrically insulated state, penetrate the inside of the tester body, and extend to the heat transfer terminal, wherein the respective end portions of the first and second heating elements, which face the contact surface of the heat transfer terminal, closely contact each other to generate thermoelectricity. Accordingly, the present invention as configured above improves treatability by effectively controlling the generated heat and accurately measuring dental pulp thermal sensitivity.

## Description

### Technical Field

The present invention relates to a thermal tester for a dental clinic, and more particularly to a thermal tester for a dental clinic that may accurately verify a thermal sensitivity of a dental pulp by rapidly transferring heat at a predetermined temperature to a tooth.

### Background Art

A pulpitis occurring in a dental pulp is caused by bacterial infection, in particular, a cavity, and includes pain. To treat the pulpitis, a treatment of soothing an inflammation and preserving the dental pulp, or removing a damaged dental pulp may be performed. The dental pulp may be damaged by an external injury of a tooth, and a treatment of the dental pulp as described above may also be required.

To perform an appropriate treatment, a degree of damage to a dental pulp needs to be accurately analyzed, prior to the above treatment of the dental pulp. However, since the dental pulp exists in a tooth, it is difficult to analyze a state of the dental pulp, before opening the tooth. Accordingly, a doctor needs to open a tooth of a patient and to expose a dental pulp of the patient when a state of the dental pulp has not been yet analyzed, which may cause an unnecessary region of the tooth to be open. Thus, there is a need for research and development of an apparatus for accurately analyzing a state of a dental pulp.

### Disclosure of Invention

### Technical Goals

An aspect of the present invention is to provide a thermal tester for a dental clinic that may accurately analyze a thermal sensitivity of a dental pulp.

Another aspect of the present invention is to provide a thermal tester for a dental clinic with an improved thermal efficiency.

### Technical solutions

According to an aspect of the present invention, there is provided a touch-sensing panel, including: a tester body; and a heating unit.

The tester body may have one side from which a heat transfer terminal protrudes and that has a contact surface that comes into contact with a tooth.

The heating unit may heat the heat transfer terminal. The heating unit may include a first heating element and a second heating element, that, in a mutually electrically insulated state, penetrate the tester body and that extend to the heat transfer terminal, and an end portion of the first heating element and an end portion of the second heating element that face the contact surface of the heat transfer terminal may be in close contact with each other, to generate a thermal electromotive force. Additionally, the heating unit may include a heat transfer element that is disposed in the heat transfer terminal to cover the first heating element and second heating element, and that transfers heat generated by the first heating element and second heating element.

Here, the heat transfer element may be formed of silver powder. A density of the heat transfer element may desirably increase, as a distance with the contact surface of the heat transfer terminal decreases. Between a diameter of an end of the heat transfer element that faces the contact surface of the heat transfer terminal, and a diameter of an opposite end of the heat transfer element that extends from the end, the diameter of the opposite end may desirably be larger than the diameter of the end.

For reference, the first heating element may include a copper wire, and the second heating element may include a resistor prepared to cover an outer circumference of the first heating element. A diameter of the second heating element may become gradually smaller toward the contact surface, within the heat transfer terminal.

The heat transfer terminal may desirably be coupled to an external fixer extending slantly from the tester body, by a coupling member formed of epoxy.

According to another aspect of the present invention, there is provided a thermal tester for a dental clinic, including: a tester body having one side from which a heat transfer terminal protrudes, the heat transfer terminal having a contact surface that comes into contact with a tooth; and a heating unit to heat the heat transfer terminal, wherein the heating unit includes a first heating element and a second heating element, that, in a mutually electrically insulated state, penetrate the tester body and that extend to the heat transfer terminal, wherein an end portion of the first heating element and an end portion of the second heating element that face the contact surface of the heat transfer terminal, are in close contact with each other, to generate a thermal electromotive force; and a heat transfer element formed of silver, and disposed in the heat transfer terminal to cover the first heating element and second heating element.

### Effect of Invention

As described above, according to the present invention, first, an end portion of a first heating element and an end portion of a second heating element that face a contact surface of a heat transfer terminal may be in close contact with each other, to generate a thermal electromotive force, and accordingly heat may be easily controlled at a specific temperature. Thus, it is possible to improve treatability.

Second, a heat transfer element formed of silver power may be included in the heat transfer terminal, and accordingly the generated thermal electromotive force may be quickly transferred to the contact surface of the heat transfer terminal. Thus, a thermal tester for a dental clinic with a reduced heat loss rate may be provided.

### Brief Description of Drawings

FIG. 1 is a side diagram schematically illustrating a thermal tester for a dental clinic according to an embodiment of the present invention,
FIG. 2 is a cross-sectional diagram schematically illustrating the thermal tester, taken along a line II-II of FIG 1, and
FIG. 3 is an enlarged cross-sectional diagram schematically illustrating a region III of FIG. 2.

### Best Mode for Carrying Out the Invention

Reference will now be made in detail to embodiments of the present invention, examples of which are illustrated in the accompanying drawings, wherein like reference numerals refer to the like elements throughout. The embodiments are described below in order to explain the present invention by referring to the figures.

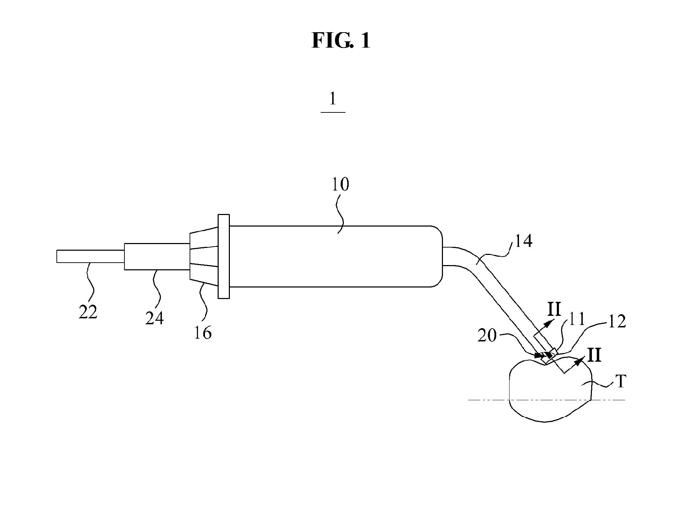

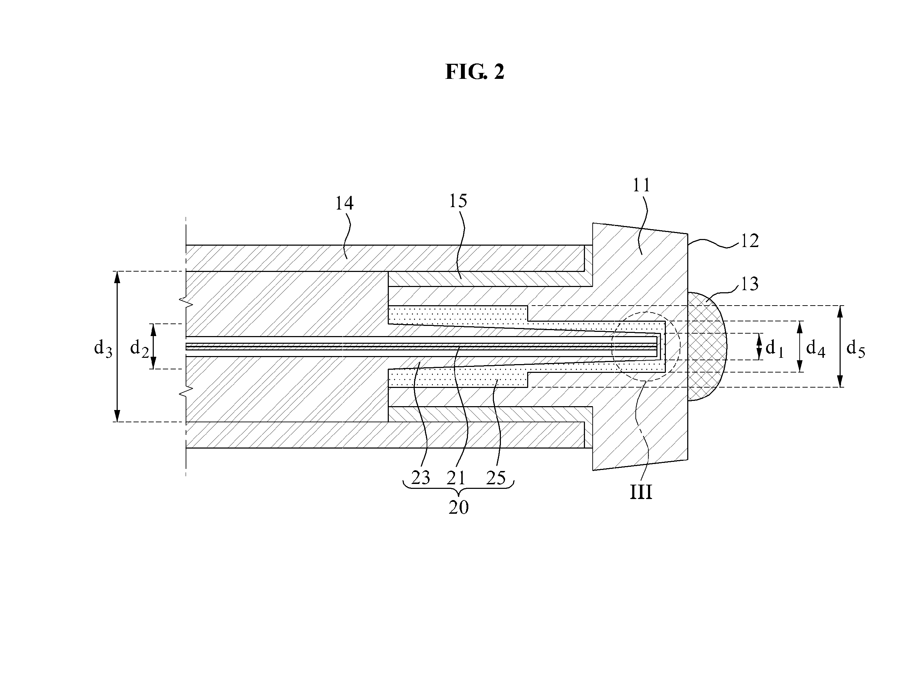

Referring to FIGS. 1 and 2, a thermal tester for a dental clinic 1 according to an embodiment of the present invention may be an apparatus for transferring heat to a tooth T of a person to be cured, testing a thermal sensitivity, namely an extent to which the person to be cured detects heat, and analyzing a degree of damaged to a nerve of the tooth T. The thermal tester 1 may include a tester body 10 and a heating unit 20.

The tester body 10 may be a body of the thermal tester 1, and may be gripped by a curer, namely a doctor. Accordingly, to protect the curer from heat, an external appearance of the tester body 10 may be insulated and protected. Additionally, a heat transfer terminal 11 may protrude from one side of the tester body 10, to transfer heat to the tooth T.

The heat transfer terminal 11 may include a contact surface 12 that comes into contact with the tooth T. The heat transfer terminal 11 may be formed of materials with an excellent heat transfer capability. A medium 13 melted by heat may be selectively attached onto the contact surface 12 of the heat transfer terminal 11, and may guide heat to the tooth T.

The heat transfer terminal 11 may be placed in an end portion of an external fixer 14 that is bent and extends from one side of the tester body 10. To facilitate an entry to the tooth T, the external fixer 14 may be bent at about 60°. The heat transfer terminal 11 may be coupled to the external fixer 14 and fixed by a coupling member 15 formed of epoxy, as shown in FIG. 2. In this instance, since the coupling member 15 is formed of epoxy, the heat transfer terminal 11 may be fixed, and external heat may be prevented from being transferred to the external fixer 14.

The heating unit 20 may heat the heat transfer terminal 11. The heating unit 20 may include a first heating element 21 and a second heating element 23 that are in close contact with each other (junction) to generate a thermal electromotive force, and a heat transfer element 25 to transfer generated heat.

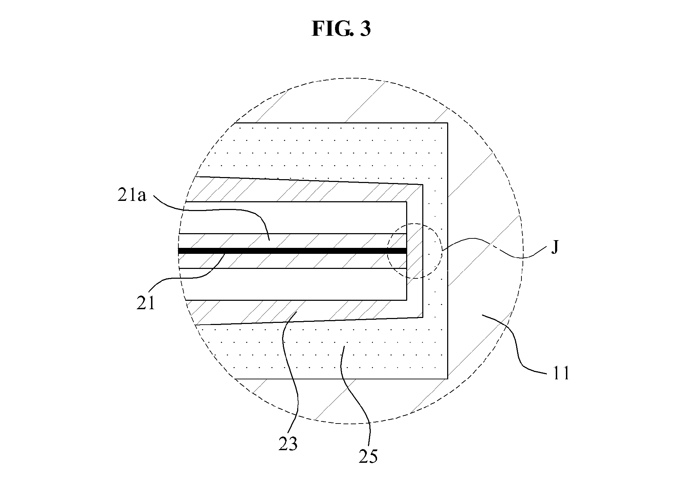

The first heating element 21 may include a copper wire that extends to the heat transfer terminal 11 through the tester body 10. As shown in FIG. 3, an outer circumference of the first heating element 21 may be coated and insulated by an insulator 21 a that is formed of enamel or ceramic materials. In this instance, an end portion of the first heating element 21 that faces the contact surface 12 of the heat transfer terminal 11 may not be coated by the insulator 21a, and may not be insulated.

The second heating element 23 may be a resistor that extends to the heat transfer terminal 11 through the tester body 10 to cover the outer circumference of the first heating element 21. In this instance, the heat transfer terminal 11 may be joined to an end portion of the first heating element 21 that is not covered by the insulator 21 a, and may form a junction portion J as shown in FIG. 3, and accordingly the thermal electromotive force may be generated. As shown in FIG. 2, a diameter of the second heating element 23 may become gradually smaller toward the contact surface 12 within the heat transfer terminal 11. Specifically, a diameter d1 of an end portion of the second heating element 23 that faces the contact surface 12 may be smaller than a diameter d2 of an opposite end portion of the heat transfer terminal 11. In this instance, an opposite end of the second heating element 23 may be enlarged and extended to have the same diameter as an internal diameter d3 of the external fixer 14. Accordingly, the second heating element 23 may be formed stepwise, so that the internal diameter d3 of the external fixer 14 may be larger than the diameter d2 of the opposite end portion of the heat transfer terminal 11.

The first heating element 21 and the second heating element 23 may be electrically connected to a first terminal 22 and a second terminal 24 that are disposed in an opposite side of the tester body 10, respectively. Here, the first terminal 22 may be formed of copper or brass, and may be insulated from the second terminal 24. The second terminal 24 may be prepared as a pipe formed of stainless steel (SUS), and may accommodate the first terminal 22. In this instance, the first terminal 22 and the second terminal 24 may be epoxy-coupled and fixed to each other, although not shown in detail. For reference, the first terminal 22 and the second terminal 24 may receive power supply from the battery, to enable the first heating element 21 and the second heating element 23 to generate heat.

The heat transfer element 25 may be prepared to cover the first heating element 21 and the second heating element 23 within the heat transfer terminal 11, and may transfer heat generated by the first heating element 21 and the second heating element 23. The heat transfer element 25 may be formed of materials with an excellent thermal conductivity, for example silver powder in the present embodiment. To effectively transfer the heat generated by the first heating element 21 and the second heating element 23 to the contact surface 12, a density of the heat transfer element 25 may increase, as a distance with the contact surface 12 decreases. Specifically, as shown in FIG. 2, between a diameter d4 of an end of the heat transfer element 25 that faces the contact surface 12 of the heat transfer terminal 11, and a diameter d5 of an opposite end extending from the end of the heat transfer element 25, the diameter d5 may have a relatively large value. In this instance, the diameter d4 of the end of the heat transfer terminal 11 may range from about 0.6 to 0.8 mm, and may be extended from about 2 to 4 mm. Additionally, the diameter d5 of the opposite end of the heat transfer terminal 11 may range from about 1.0 to 1.3 mm, and may be extended from about 2 to 3 mm. Since the diameter d4 of the end of the heat transfer terminal 11 is smaller than the diameter d5 of the opposite end, an amount of the silver powder may increase from the end to the opposite end, and instead, a density may be reduced. Accordingly, in a state in which an energy loss of heat generated in the junction portion J is reduced as much as possible, the heat may be rapidly transferred to the contact surface 12 of the heat transfer terminal 11.

An operation of the thermal tester 1 configured as described above will be described with reference to FIGS. 1 through 3.

Referring to FIG. 1, the thermal tester 1 may enter the tooth T, and the contact surface 12 of the heat transfer terminal 11 may come in contact with the tooth T. In this instance, the medium 13 melted by heat may be in contact with the tooth T in the contact surface 12, and may transfer heat. The heat transferred through the contact surface 12 may be generated by the thermal electromotive force by the junction portion J formed by an end portion of each of the first heating element 21 and the second heating element 23 that are electrically connected to the first terminal 22 and the second terminal 24, as shown in FIGS. 2 and 3. The heat generated by the junction portion J may be rapidly diffused through the heat transfer element 25 formed of silver power, and may be transferred to the contact surface 12. When heat at a specific temperature, for example a temperature of 150°C to 200°C, is transferred to the tooth T through the contact surface 12, a curer may verify a degree of damage to a dental pulp, by analyzing an extent to which a person to be cured detects the heat.

Although a few embodiments of the present invention have been shown and described, the present invention is not limited to the described embodiments. Instead, it would be appreciated by those skilled in the art that changes may be made to these embodiments without departing from the principles and spirit of the invention, the scope of which is defined by the claims and their equivalents.

## Claims

1. A thermal tester for a dental clinic, comprising:
a tester body having one side from which a heat transfer terminal protrudes, the heat transfer terminal having a contact surface that comes into contact with a tooth; and
a heating unit to heat the heat transfer terminal,
wherein the heating unit comprises a first heating element and a second heating element, that, in a mutually electrically insulated state, penetrate the tester body and that extend to the heat transfer terminal, wherein an end portion of the first heating element and an end portion of the second heating element that face the contact surface of the heat transfer terminal, are in close contact with each other, to generate a thermal electromotive force.

2. The thermal tester of claim 1, wherein the heating unit comprises a heat transfer element that is disposed in the heat transfer terminal to cover the first heating element and second heating element, and that transfers heat generated by the first heating element and second heating element.

3. The thermal tester of claim 2, wherein the heat transfer element is formed of silver powder.

4. The thermal tester of claim 3, wherein a density of the heat transfer element increases, as a distance with the contact surface of the heat transfer terminal decreases.

5. The thermal tester of claim 4, wherein, between a diameter of an end of the heat transfer element that faces the contact surface of the heat transfer terminal, and a diameter of an opposite end of the heat transfer element that extends from the end, the diameter of the opposite end is larger than the diameter of the end.

6. The thermal tester of one of claim 1, wherein the first heating element comprises a copper wire, and the second heating element comprises a resistor prepared to cover an outer circumference of the first heating element, and
wherein a diameter of the second heating element becomes gradually smaller toward the contact surface, within the heat transfer terminal.

7. The thermal tester of claim l, wherein the heat transfer terminal is coupled to an external fixer extending slantly from the tester body, by a coupling member formed of epoxy.

8. A thermal tester for a dental clinic, comprising:
a tester body having one side from which a heat transfer terminal protrudes, an external appearance of the tester body being insulated, and the heat transfer terminal having a contact surface that comes into contact with a tooth; and
a heating unit to heat the heat transfer terminal,
wherein the heating unit comprises:
a first heating element and a second heating element, that, in a mutually electrically insulated state, penetrate the tester body and that extend to the heat transfer terminal, wherein an end portion of the first heating element and an end portion of the second heating element that face the contact surface of the heat transfer terminal, are in close contact with each other, to generate a thermal electromotive force; and
a heat transfer element formed of silver, and disposed in the heat transfer terminal to cover the first heating element and second heating element.

9. The thermal tester of claim 8, wherein a density of the heat transfer element increases, as a distance with the contact surface of the heat transfer terminal decreases.
